# EUROPEAN PATENT SPECIFICATION

(11) **EP 0 805 133 B1**
(45) Date of publication and mention of the grant of the patent: **07.10.1998**
(21) Application number: 97201280.1
(22) Date of filing: 28.04.1997
(51) Int. Cl.: C07C 2/86, C07C 13/15

(54) **Process for cyclopentadiene substitution**
Verfahren zur Substitution von Cyclopentadien
Procédé de substitution de cyclopentadiène

(30) Priority: 03.05.1996 NL 1002998
(43) Date of publication of application: 05.11.1997
(73) Proprietor: DSM N.V., 6411 TE Heerlen (NL)
(72) Inventor: van Beek, Johannes Antonius Maria, Mountain View, CA 94043 (US); Gruter, Gerardus Johannes Maria, 6217 LW Maastricht (NL); Green, Richard, 6161 TK Geleen (NL)

(56) References cited:
- JOURNAL OF THE AMERICAN CHEMICAL SOCIETY, vol. 113, no. 13, 19 June 1991, DC US, pages 4843-4851, XP002021659 R. ALLEN WILLIAMS ET AL. : "Encapsulated Alkaline-Earth Metallocenes. Synthesis, Solution, Behavior, and Solid-State Structures of Bis(tetraisopropylcyclopentadienyl)calcium and -barium, [(C3H7)4C5]2Ca and [(C3H7)4C5H]2Ba"
- JOURNAL OF THE AMERICAN CHEMICAL SOCIETY, vol. 112, no. 7, 28 March 1990, DC US, pages 2808-2809, XP002021660 CLIFFORD G. VENIER ET AL.: "D-tert-butylcyclopentadiene and Tri-tert-butylcyclopentadiene"
- TETRAHEDRON LETTERS, vol. 32, no. 41, 7 October 1991, OXFORD GB, pages 5773-5776, XP002021661 ECKEHARD V. DEHMLOW ET AL.: "Phase Transfer Catalyzed Tert-Alkylations of Cyclopentadiene and Indene: Indications for Set Processes"

## Description

The invention relates to a process for the preparation of a substituted cyclopentadiene compound, which process consists of reacting a halogenide of the substituting compound in a mixture of the cyclopentadiene compound and an aqueous solution of a base in the presence of a phase transfer catalyst.

In the following the abbreviation 'Cp' will be used for cyclopentadiene. The same abbreviation will be used for a cyclopentadienyl group if it is clear from the context whether cyclopentadiene itself or its anion is meant.

Such a process is known from the Journal of the American Chemical Society, 1991, 113, 4843-4851, which describes the preparation of Cp's substituted with isopropyl groups in a mixture of aqueous KOH, isopropyl bromide and Cp in a molar ratio of 40:5:1, Adogen 464 being used as phase transfer catalyst.

A drawback of the known process is that in this case a mixture of tri- and tetraisopropyl Cp in a ratio of 65:35 is obtained, which was split up in an additional step in order to obtain the individual components in a more pure state.

The object of the invention is a process for the preparation of substituted Cp compounds with a greater selectivity than the known process.

This object is achieved according to the invention in that during the reaction the quantity of base relative to the Cp compound is at any moment between 5 and 30 mol/mol.

With the process according to the invention, substituted Cp compounds are obtained which have the desired degree of substitution. These Cp compounds are obtained with a higher selectivity than with the known process.

From J. Am. Chem. Soc., 1990, 112, 2808-2809, it is known to prepare di-t-butyl Cp with a selectivity of 90%, but such a high selectivity is an exception, as appears from the publication referred to above. The high selectivity in this case is bound up with the circumstance that the substituting compound is a tertiary alkyl. During the reaction there is a high degree of steric hindrance and it is in fact the low probability of obtaining the triply substituted compound which is responsible for the high percentage of di-substituted compounds. This selection mechanism will not be effective in the case of substituents which are subject to less steric hindrance, for instance in the case of n- or sec-alkyl groups.

By Cp compounds are understood Cp as such and Cp which is already substituted, with the possibility of two substituents forming a closed ring. The process according to the invention thus enables unsubstituted compounds to be converted to singly or multiply substituted ones, but also singly or multiply substituted Cp compounds to be substituted further. During substitution ring closure can occur.

Depending on the size and the related degree of steric hindrance of the compounds to be substituted, three- to sixfold substituted compounds can be obtained. Tertiary butyl and other tertiary alkyls are examples of substituents which by the process according to the invention under practical conditions (1-10 bar, -20 to 120°C) can be substituted up to threefold on a Cp molecule, while primary and secondary alkyl halides can be substituted on a Cp-molecule in general up to fourfold and mostly even five- or sixfold under these mild conditions.

Suitable substituents are for example alkyl groups, linear as well as branched and cyclic ones, alkenyl and aralkyl groups. Further, these may also contains, besides carbon and hydrogen, one or more hetero atoms from groups 14-17 of the Periodic System, for instance O, N, Si or F. The substituents are used in the process in the form of their halides, by preference in the form of their bromides. When bromides are used it appears that a smaller amount of phase transfer catalyst suffices and that a greater yield of the desired compound is achieved. Examples of suitable substituting groups are methyl, ethyl, (iso)propyl, secondary butyl, pentyl, hexyl and octyl, (tertiary) butyl and other homologues, cyclohexyl, benzyl.

A further advantage of the process according to the invention lies in the fact that a virtually equivalent amount of the halogenated substituting compound with respect to the Cp-compound can be used, so that in the final reaction mixture a smaller amount of non-reacted halogenide remains behind. An equivalent amount is understood to be an amount in moles which corresponds to the desired multiplicity of substitution, for instance 2 mol per mol of Cp compound if twofold substitution with the substituent involved is desired. In contrast, in the above-mentioned publication in the Journal of the American Chemical Society, 1991, 113, 4843-4851, where 5 equivalents of isopropyl bromide have to be added in order to obtain merely a mixture of three- and fourfold substituted Cp compounds. In particular if primary or secondary alkyl bromides are substituted it is preferable to use these in equivalent amounts or in an excess of at most 20% but preferably at most 10%. If due to steric hindrance incorporation in excess of the desired amount is improbable or even impossible anyway, the amount of substituting compound present in the reaction mixture is less relevant. This applies in particular in the case of substitution of tertiary alkyls or other voluminous substituents and in the case of substitution of the fourth, fifth or sixth substituent on the same Cp molecule.

Owing to the high degree of selectivity of the process according to the invention it permits obtaining, without intermediate separation or purification, Cp compounds which are substituted with specific combinations of substituents. It is thus possible for instance first to effect a twofold substitution by means of a certain halide, whereafter in the same reaction mixture a third substitution can occur by adding a second, other halide of a substituting compound to the mixture after some time. This can be repeated, so that the process according to the invention also enables Cp derivatives with three or more different substituents to be obtained.

The process according to the invention offers an improved selectivity relating to the substitution degree of the Cp compounds. If linear alkyls are substituted, position isomers may form as a result of competition of 1,2- and 1,3-substitution. In the case of substitution with secondary or tertiary halides of substituting compounds a second substituent will in general not be substituted in a position adjacent to the first substituent (position isomerism). Several double bond isomers are formed per position isomer. When used as a ligand in a metal complex the distinction between double bond isomers does not play a role any more. Separation of those is therefore superfluous. The foregoing applies by analogy to triply substituted compounds. Geminal substitution may also occur. During a geminal substitution the number of substituents increases with 1, but the number of substituted carbon atoms does not increase. Geminally substituted Cp compounds can easily be separated from the non-geminally substituted ones, because the latter can be converted to a Cp-anion, in contrast to the former. All these forms of isomerism also occur in the known process, but the additional occurrence of different degrees of substitution in that process makes splitting up into the different components much more difficult than in the process according to the invention.

The substitution takes place in a mixture of the Cp compound and an aqueous solution of a base. The concentration of the base in the solution is preferably between 20 and 80 wt.%, more preferably between 40 and 60 wt.%. Concentrations of about 50 wt.% have been found to be most suitable.

By preference a hydroxide of an alkali metal, for instance K or Na, is used as a base. It has been found that the use of NaOH instead of KOH which is commonly used in the state of the art results in a considerable increase in the rate of reaction, which is the reason why NaOH is preferably used as base. The base is present in an amount of 5-30 mol per mole of Cp compound, preferably in an amount of 6-20, more preferably in an amount of 7-15 mol per mole of Cp compound. These quantities are significantly lower than the amount of 40 mol per mole of Cp compound as usual in the state of the art.

It has appeared that a substantial reduction of the reaction time can be achieved if the solution of the base is refreshed during the reaction, for instance by first mixing the solution of the base with the other components of the reaction mixture and after some time isolating the aqueous phase and replacing it by a fresh portion of solution of the base. The substitution takes place at atmospheric or elevated pressure, for instance up to 100 MPa, which higher level is applied in particular if volatile components are present. The temperature at which the reaction takes place may vary within wide limits, for instance from -20 to 120°C, preferably between 10 and 50°C. Starting up the reaction at room temperature is usually a suitable step, after which the temperature of the reaction mixture can rise due to the heat released in the reaction.

The substitution takes place in the presence of a phase transfer catalyst which is able to transfer OH-ions from the aqueous phase to the organic phase containing Cp compound and the substituting compound. The OH-ions react in the organic phase with a H-atom which can be split off from the Cp compound. Possible phase transfer catalysts to be used are quaternary ammonium, phosphonium, arsonium, stibonium, bismuthonium, and tertiary sulphonium salts. More preferably, ammonium and phosphonium salts are used, for example tricaprylmethylammonium chloride, commercially available under the name Aliquat 336 (Fluka AG, Switzerland; General Mills Co., USA) and Adogen 464 (Aldrich Chemical Co., USA). Compounds such as benzyltriethylammonium chloride (TEBA) or benzyltriethylammonium bromide (TEBA-Br), benzyltrimethylammonium chloride, benzyltrimethylammonium bromide or benzyltrimethylammonium hydroxide (Triton B), tetra-n-butylammonium chloride, tetra-n-butylammonium bromide, tetra-n-butylammonium iodide, tetra-n-butylammonium hydrogen sulphate or tetra-n-butylammonium hydroxide and cetyltrimethylammonium bromide or cetyltrimethylammonium chloride, benzyltributyl-, tetra-n-pentyl-, tetra-n-hexyl- and trioctylpropylammonium chlorides and their bromides are likewise suitable. Usable phosphonium salts include, for example, tributylhexadecylphosphonium bromide, ethyltriphenylphosphonium bromide, tetraphenylphosphonium chloride, benzyltriphenylphosphonium iodide and tetrabutylphosphonium chloride. Crown ethers and cryptands can also be used as a phase transfer catalyst, for example 15-crown-5, 18-crown-6, dibenzo-18-crown-6, dicyclohexano-18-crown-6, 4,7,13,16,21-pentaoxa-1,10-diazabicyclo[8.8.5]tricosane (Kryptofix 221), 4,7,13,18-tetraoxa-1,10-diazabicyclo[8.5.5]eicosane (Kryptofix 211) and 4,7,13,16,21,24-hexaoxa-1,10-diazabicyclo[8.8.8]-hexacosane ("[2.2.2]") and its benzo derivative Kryptofix 222 B. Polyethers such as ethers of ethylene glycols can also be used as a phase transfer catalyst. Quaternary ammonium salts, phosphonium salts, phosphoric acid triamides, crown ethers, polyethers and cryptands can also be used on supports such as, for example, on a crosslinked polystyrene or another polymer. The phase transfer catalyst is used in an amount of 0.01 - 2, preferably 0.05 - 1 equivalents on the basis of the amount of Cp-compound.

The sequence of addition of the various components to the reactor as a rule is not essential in the implementation of the process. A suitable procedure is first to add the solution of the base, catalyst and Cp compound and then, after thorough stirring of these, the halide of the substituting compound is added. It is also possible to first add and thoroughly stir the halide of the substituting compound, the Cp compound and the catalyst and then add the solution of the base. In all the embodiments described it is found that a considerable shortening of the reaction time can be achieved if the solution of the base is removed from the reaction mixture after some time and replaced by a fresh portion of the solution of the base. This appears to be advantageous in particular when a tri- or tetrasubstitution is carried out. A further advantage of intermediate refreshing of the aqueous phase, besides the shortening of the reaction time, is that a much smaller reaction volume suffices. This advantage is achieved owing to the amount of aqueous phase as such being much smaller as well than in the process according to the state of the art. Optionally, at the same time as the solution of the base is replaced a following desired substituent in halogenated form can be added in order to obtain a Cp compound substituted with different substituents as described in the foregoing. The timing of replacement of the solution of the base and/or addition of any second substituting compound can be determined by monitoring the reaction in time with gas chromatography. The decrease of the rate of reaction determines the timing of replacement of the solution of the base. With gas chromatography the timing of addition of any second halide of a substituting compound can also be determined.

The order of addition of the substituting compounds may influence the identity of the position isomer which is obtained.

Upon completion of the reaction the aqueous phase and the organic phase containing the Cp compound are separated. When necessary, the Cp compound is recovered by fractionated distillation.

Cp-containing compounds are for instance used as ligands in metal complexes which are used as catalysts for the polymerisation of olefins. Because it cannot be predicted directly on the basis of the structure of a Cp-containing compound whether, when incorporated in a metal complex, it will have a beneficial effect on the catalytic properties of that particular complex, it is important to obtain as many different Cp compounds as possible. The invention thus fills a clear need.

The invention is illustrated by the following non-restrictive examples. The following analytical methods were used for characterization. Gas chromatography (GC) was carried out on a Hewlett-Packard 5890 series II with an HP crosslinked methyl silicon gum (25 m × 0.32 mm × 1.05 µm) column. Combined gas chromatography/ mass spectrometry (GC-MS) was carried out with a Fisons MD800 equipped with a quadrupole mass detector, autoinjector Fisons AS800 and CPSil8 column (30 m × 0.25 mm × 1 µm, low bleed). NMR was carried out on a Bruker ACP200 (¹H=200 MHz; ¹³C=50 MHz) or a Bruker ARX400 (¹H=400 MHz; ¹³C=100 MHz). To characterize metal complexes, of a Kratos MS80 or alternatively a Finnigan Mat 4610 mass spectrometer was used.

### Example I

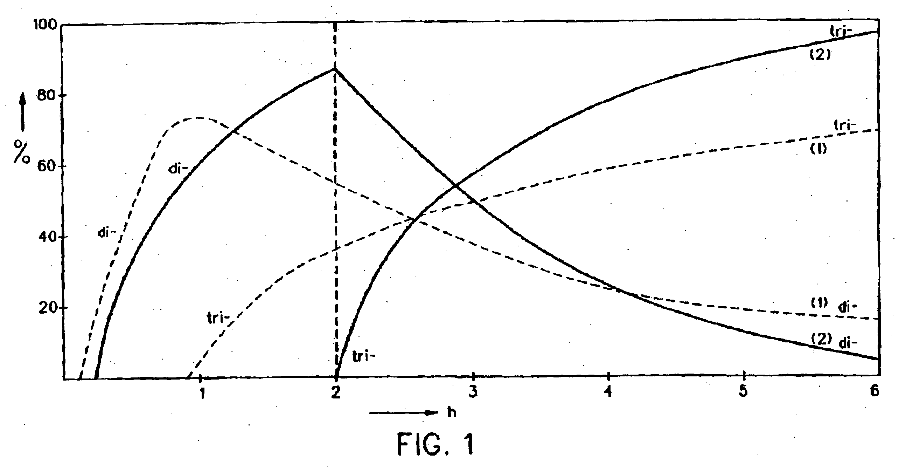

Two reactions carried out according to the process of the invention were monitored in time using gas chromatography. The results are presented in Fig. 1. The percentages (%) of di- and tri-substituted cyclopentadiene compounds formed relative to the total of cyclopentadiene compounds were plotted against the reaction time (h) for both reactions in figure 1. In reaction 1 (dashed curves in figure 1) the process according to the invention was carried out with a certain amount of cyclopentadiene + 3 equivalents of isopropylbromide + 10 mol% of Aliquat in 20 equivalents of 50% sodium hydroxide in water. In reaction 2 (solid curves in figure 1) the ratios were: 1 equivalent of cyclopentadiene + 3 equivalents of isopropylbromide + 10 mol% of Aliquat in 10 equivalents of sodium hydroxide (50%). In this case, in deviation from reaction 1, the stirring was stopped after 2 hours and after phase separation the layer of water was drawn off, after which 10 equivalents of sodium hydroxide (50% in water) were added again. Then stirring was continued for another 4 hours. So in all the same number of equivalents of NaOH was used in the two reactions.

From figure 1 it appears that great differences occur in the development of the two reactions. In reaction 1 a mixture of di- and triisopropylcyclopentadiene in a ratio of 21 : 68 is present after 6 hours' stirring. When stirring is continued the amount of diisopropylcyclopentadiene will decrease and the amount of triisopropylcyclopentadiene will increase correspondingly. In reaction 2 both absolutely and relatively much more triisopropylcyclopentadiene is present after 6 hours' stirring. The ratio between tri- and diisopropylcyclopentadiene is 93 : 4 in this case. The figure also shows the higher selectivity for the reaction with less base (reaction 2). After 2 hours there is still no triisopropylcyclopentadiene present in reaction 2, while at that moment already 85% of diisopropylcyclopentadiene is present. In reaction 1 there is already formation of triisopropylcyclopentadiene as soon as only 70% of diisopropylcyclopentadiene has formed. This shows the favourable effect of lowering of the amounts of equivalents of hydroxide on the selectivity towards the number of substituted groups. It may be observed that from the trend of the solid curve it is very likely that the amount of diisopropylcyclopentadiene will further increase if the reaction is allowed to continue. In this example the increase turns into an abrupt decline as a result of the replacement of the base-containing water layer. It appears that this triggers off the formation of triisopropylcyclopentadiene. This means that due to appropriate timing of replacement of the water layer both the reaction rate and the nature of the reaction product can be influenced. In reaction 1 as well as in reaction 2 triisopropylcyclopentadiene is ultimately obtained with a higher selectivity than with the process according to the state of the art, in which the reaction is carried out in the presence of 40 equivalents of hydroxide.

### Example II

### Preparation of di(2-propyl)cyclopentadiene

A double-walled reactor having a volume of 200 ml, provided with baffles, condenser, top stirrer, thermometer and dropping funnel was charged with 180 g of clear 50% NaOH (2.25 mol), 9.5 g of Aliquat 336 (23 mmol) and 15 g (0.227 mol) of freshly cracked cyclopentadiene. The reaction mixture was stirred turbulently for a few minutes. Then 56 g of 2-propyl bromide (1.05 mol) were added, cooling with water taking place at the same time. A few minutes after the addition of the 2-propyl bromide the temperature rose by about 10°C. This was followed by 6 hours' stirring at 50°C. GC was used to show that at that instant 92% of di(2-propyl)cyclopentadiene was present in the mixture of di- and tri(2-propyl)cyclopentadiene. The product was distilled at 10 mbar and 70°C. After distillation, 25.35 g of di(2-propyl)cyclopentadiene were obtained. Characterization took place with the aid of GC, GC-MS, ¹³C- and ¹H-NMR.

### Example III

### Preparation of tri(2-propyl)cyclopentadiene

A double-walled reactor having a volume of 200 ml, provided with baffles, condenser, top stirrer, thermometer and dropping funnel was charged with 180 g (2.25 mol) of clear 50% NaOH, 9.5 g of Aliquat 336 (23 mmol) and 15 g (0.227 mol) of freshly cracked cyclopentadiene were added. The reaction mixture was stirred turbulently for a few minutes. Then 84 g of 2-propyl bromide (0.68 mol) were added with simultaneous cooling with water. A few minutes after the addition of the 2-propyl bromide the temperature rose by about 10°C. GC was used to show that about 30 minutes after all 2-propyl bromide had been added, (monosubstituted) 2-propylcyclopentadiene had formed. Then the mixture was heated to 50°C. After 2 hours the stirring was stopped and phase separation was awaited. The water layer was drawn off and 180 g (2.25 mol) of fresh 50% strength NaOH were added, followed by a further 1 hour's stirring at 50°C. GC was used to show that at that instant the mixture of di-, tri- and tetracyclopentadiene contained 90 to 95% of tri(2-propyl)cyclopentadiene. The product was distilled at 1.3 mbar and 77-78°C. After distillation, 31.9 g of tri(2-propyl)cyclopentadiene were obtained. Characterization took place with the aid of GC, GC-MS, ¹³C- and ¹H-NMR.

### Example IV

### Preparation of tetra(2-propyl)cyclopentadiene

Analogously to Example III, 114 g of 2-propyl bromide (0.93 mol) was added and the water layer was replaced for a second time after 7 hours and at the same time another 5 g (12 mmol) of Aliquat 336 were added. Then heating for 16 hours at 55°C took place. GC showed that at that instant 85% of tetra(2-propyl)cyclopentadiene was present in the mixture of tri- and tetracyclopentadiene. The product was distilled at 1.0 mbar and 88-90°C. After distillation, 34.9 g of tetra(2-propyl)cyclopentadiene were obtained. Characterization took place with the aid of GC, GC-MS, ¹³C- and ¹H-NMR.

### Example V

### Preparation of di(cyclohexyl)cyclopentadiene

A double-walled reactor having a volume of 1 L, provided with baffles, condenser, top stirrer, thermometer and dropping funnel was charged with 600 g of clear 50% NaOH (7.5 mol), followed by cooling to 8°C. Then 20 g of Aliquat 336 (49 mmol) and 33 g (0.5 mol) of freshly cracked cyclopentadiene were added. The reaction mixture was stirred turbulently for a few minutes. Then 172 g of cyclohexyl bromide (1.05 mol) were added, cooling with water taking place at the same time. After 2 hours' stirring at room temperature the reaction mixture was heated to 70°C, followed by a further 6 hours' stirring. GC was used to show that at that instant 79% of di(cyclohexyl)cyclopentadiene were present. The product was distilled at 0.04 mbar and 110-120°C. After distillation, 73.6 g of di(cyclohexyl)cyclopentadiene were obtained. Characterization took place with the aid of GC, GC-MS, ¹³C- and ¹H-NMR.

### Example VI

### Preparation of tri(cyclohexyl)cyclopentadiene

A double-walled reactor having a volume of 1 L, provided with baffles, condenser, top stirrer, thermometer and dropping funnel was charged with 600 g of clear 50% NaOH (7.5 mol), followed by cooling to 8°C. Then 20 g of Aliquat 336 (49 mmol) and 33 g (0.5 mol) of freshly cracked cyclopentadiene were added. The reaction mixture was stirred turbulently for a few minutes. Then 256 g of cyclohexyl bromide (1.57 mol) were added, cooling with water taking place at the same time. After 1 hour's stirring at room temperature the reaction mixture was heated to 70°C, followed by a further 2 hours' stirring. After 2 hours, stirring was stopped and phase separation was awaited. The water layer was drawn off and 600 g (7.5 mol) of fresh 50% strength NaOH were added, followed by a further 4 hours' stirring at 70°C. GC was used to show that at that instant 10% di- and 90% tri(cyclohexyl)cyclopentadiene were present in the mixture. The product was distilled at 0.04 mbar and 130°C. After distillation, 87.4 g of tri(cyclohexyl)cyclopentadiene were obtained. Characterization took place with the aid of GC, GC-MS, ¹³C- and ¹H-NMR.

### Example VII

### Preparation of tetra(ethyl)cyclopentadiene

A double-walled reactor having a volume of 1 L, provided with baffles, condenser, top stirrer, thermometer and dropping funnel was charged with 1050 g of clear 50% NaOH (13.1 mol), followed by cooling to 10°C. Then 32 g of Aliquat 336 (79 mmol) and 51 g (0.77 mol) of freshly cracked cyclopentadiene were added. The reaction mixture was stirred turbulently for a few minutes. Then 344 g of ethyl bromide (3.19 mol) were added gradually in one hour's time, cooling with water taking place at the same time. After 1 hour's stirring at room temperature the reaction mixture was heated to 35°C, followed by a further 6 hours' stirring. Stirring was stopped and phase separation was awaited. The water layer was drawn off and 1050 g (13.1 mol) of fresh 50% strength NaOH were added, followed by a further 5 hours' stirring at room temperature. GC was used to show that at that instant 15% tri-, 78% tetra- and 7% of penta(ethyl)cyclopentadiene were present in the mixture. The product was distilled at 11 mbar and 91°C. After distillation, 74.8 g of tetra(ethyl)cyclopentadiene were obtained. Characterization took place with the aid of GC, GC-MS, ¹³C- and ¹H-NMR.

### Example VIII

### Preparation of di(2-butyl)cyclopentadiene

A double-walled reactor having a volume of 1 L, provided with baffles, condenser, top stirrer, thermometer and dropping funnel was charged with 600 g of clear 50% NaOH (7.5 mol), followed by cooling to 10°C. Then 30 g of Aliquat 336 (74 mmol) and 48.2 g (0.73 mol) of freshly cracked cyclopentadiene were added. The reaction mixture was stirred turbulently for a few minutes. Then 200 g of 2-butyl bromide (1.46 mol) were added gradually in half an hour's time, cooling with water taking place at the same time. After 2 hours' stirring at room temperature the reaction mixture was heated to 60°C, followed by a further 4 hours' stirring. GC was used to show that at that instant more than 90% of di(2-butyl)cyclopentadiene was present in the mixture. The product was distilled at 20 mbar and 80-90°C. After distillation, 90.8 g of di(2-butyl)cyclopentadiene were obtained. Characterization took place with the aid of GC, GC-MS, ¹³C- and ¹H-NMR.

### Example IX

### Preparation of tri(2-butyl)cyclopentadiene

A double-walled reactor having a volume of 1 L, provided with baffles, condenser, top stirrer, thermometer and dropping funnel was charged with 400 g of clear 50% NaOH (5 mol). Then 9.6 g of Aliquat 336 (24 mmol) and 15.2 g (0.23 mol) of freshly cracked cyclopentadiene were added. The reaction mixture was stirred turbulently for a few minutes. Then 99.8 g of 2-butyl bromide (0.73 mol) were added in half an hour's time, cooling with water taking place at the same time. After half an hour's stirring at room temperature the reaction mixture was heated to 70°C, followed by a further 3 hours' stirring. Stirring was stopped and phase separation was awaited. The water layer was drawn off and 400 g (5.0 mol) of fresh 50% strength NaOH were added, followed by a further 2 hours' stirring at 70°C. GC was used to show that at that instant more than 90% of tri(2-butyl)cyclopentadiene was present in the mixture of di-, tri- and tetra(2-butyl)cyclopentadiene. The product was distilled at 1 mbar and 91°C. After distillation, 40.9 g of tri(2-butyl)cyclopentadiene were obtained. Characterization took place with the aid of GC, GC-MS, ¹³C- and ¹H-NMR.

### Example X

### Preparation of di- and tri(2-pentyl)cyclopentadiene

A double-walled reactor having a volume of 1 L, provided with baffles, condenser, top stirrer, thermometer and dropping funnel was charged with 900 g (11.25 mol) of clear 50% NaOH. Then 31 g of Aliquat 336 (77 mmol) and 26.8 g (0.41 mol) of freshly cracked cyclopentadiene were added. The reaction mixture was stirred turbulently for a few minutes. Then 155 g of 2-pentyl bromide (1.03 mol) were added over a period of 1 hour, cooling with water taking place at the same time. After 3 hours' stirring at room temperature the reaction mixture was heated to 70°C, followed by a further 2 hours' stirring. Stirring was stopped and phase separation was awaited. The water layer was drawn off and 900 g (11.25 mol) of fresh 50% strength NaOH were added, followed by a further two hours' stirring at 70°C. GC was used to show that at that instant the mixture consisted of di- and tri(2-pentyl)cyclopentadiene (approximately 1 : 1). The products were distilled at 2 mbar, 79-81°C and 0.5 mbar, 102°C, respectively. After distillation, 28 g of di- and 40 g of tri(2-pentyl)cyclopentadiene were obtained. Characterization took place with the aid of GC, GC-MS, ¹³C- and ¹H-NMR.

### Example XI

### Preparation of di- and tri(3-pentyl)cyclopentadiene

A double-walled reactor having a volume of 1 L, provided with baffles, condenser, top stirrer, thermometer and dropping funnel was charged with 430 g (5.4 mol) of clear 50% NaOH. Then 23 g of Aliquat 336 (57 mmol) and 27 g (0.41 mol) of freshly cracked cyclopentadiene were added. The reaction mixture was stirred turbulently for a few minutes. Then 150 g of 3-pentyl bromide (1.0 mol) were added over a period of 1 hour, cooling with water taking place at the same time. After 1 hour's stirring at room temperature the reaction mixture was heated to 70°C, followed by a further 3 hours' stirring. Stirring was stopped and phase separation was awaited. The water layer was drawn off and 540 g (6.70 mol) of fresh 50% strength NaOH were added, followed by a further four hours' stirring at 70°C. GC was used to show that at that instant the mixture consisted of di- and tri(3-pentyl)cyclopentadiene (approximately 3 : 2). The products were distilled at 0.2 mbar, 51°C and 0.2 mbar, 77-80°C, respectively. After distillation, 32 g of di- and 18 g of tri(3-pentyl)cyclopentadiene were obtained. Characterization took place with the aid of GC, GC-MS, ¹³C- and ¹H-NMR.

### Example XII

### Preparation of di(2-propyl)cyclohexylcyclopentadiene

In a double-walled reactor having a volume of 200 mL, provided with baffles, condenser, top stirrer, thermometer and dropping funnel, 150 g of clear 50% NaOH (1.9 mol), 7 g of Aliquat 336 (17.3 mmol) and 8.5 g (0.13 mol) of freshly cracked cyclopentadiene were combined. The reaction mixture was stirred turbulently for a few minutes. Then 31.5 g of 2-propyl bromide (0.26 mol) were added, cooling with water taking place at the same time. Metering in took a total time of 1 hour. After addition of the bromide the reaction mixture was heated to 50°C. After 2 hours, stirring was stopped and phase separation was awaited. The water layer was drawn off, and 150 g (1.9 mol) of fresh 50% strength NaOH were added. This was followed by the addition of 20.9 g (0.13 mol) of cyclohexyl bromide, and stirring was then continued for a further 3 hours at 70°C. GC was used to show that at that instant 80% of di(2-propyl)cyclohexylcyclopentadiene was present in the mixture. The product was distilled at 0.3 mbar and 80°C. After distillation, 17.8 g of di(2-propyl)cyclohexylcyclopentadiene were obtained. Characterization took place with the aid of GC, GC-MS, ¹³C- and ¹H-NMR.

### Example XIII

### a. Preparation of tetra(octyl)cyclopentadiene

A double-walled reactor having a volume of 1.5 L, provided with baffles, condenser, top stirrer, thermometer and dropping funnel was charged with 900 g of clear 50% NaOH (11.3 mol), followed by cooling to 10°C. Then 30 g of Aliquat 336 (74 mmol) and 48 g (0.72 mol) of freshly cracked cyclopentadiene were added. The reaction mixture was stirred turbulently for a few minutes. Then 577 g of octyl bromide (2.99 mol) were added in one hour's time, cooling with water taking place at the same time. After 1 hour's stirring at room temperature the reaction mixture was heated to 35°C, followed by a further 6 hours' stirring. Stirring was stopped and phase separation was awaited. The water layer was drawn off and 920 g (11.5 mol) of fresh 50% strength NaOH were added, followed by a further 5 hours' stirring at room temperature. GC was used to show that at that instant 10% of tri-, 83% of tetra- and 7% of penta(octyl)cyclopentadiene were present in the mixture. The product was distilled at reduced pressure. After vacuum distillation, 226.6 g of tetra(octyl)cyclopentadiene were obtained. Characterization of the product took place with the aid of GC, GC-MS, ¹³C- and ¹H-NMR.

### Example XIV

### a. Preparation of tetra(n-propyl)cyclopentadiene

A double-walled reactor having a volume of 1 L, provided with baffles, condenser, top stirrer, thermometer and dropping funnel was charged with 1000 g of clear 50% NaOH (12.5 mol), followed by cooling to 10°C. Then 30 g of Aliquat 336 (74 mmol) and 50 g (0.75 mol) of freshly cracked cyclopentadiene were added. The reaction mixture was stirred turbulently for a few minutes. Then 373 g of propyl bromide (3.03 mol) were added in one hour's time, cooling with water taking place at the same time. After 1 hour's stirring at room temperature the reaction mixture was heated to 35°C, followed by a further 6 hours' stirring. Stirring was stopped and phase separation was awaited. The water layer was drawn off and 990 g (12.4 mol) of fresh 50% strength NaOH were added, followed by a further 5 hours' stirring at room temperature. GC was used to show that at that instant 14% of tri-, 80% of tetra- and 6% of penta(propyl)cyclopentadiene were present in the mixture. The product was distilled at reduced pressure. After vacuum distillation, 103.1 g of tetra(propyl)cyclopentadiene were obtained. Characterization of the product took place with the aid of GC, GC-MS, ¹³C- and ¹H-NMR.

## Claims

1. Process for the preparation of a substituted cyclopentadiene compound, which process consists of reacting a halide of a substituting compound in a mixture of the cyclopentadiene compound and an aqueous solution of a base in the presence of a phase transfer catalyst, characterized in that during the reaction the quantity of base relative to the cyclopentadiene compound is at any moment between 5 and 30 mol/mol.

2. Process according to Claim 1, wherein the quantity of base relative to the cyclopentadiene compound is between 7 and 15 mol/mol.

3. Process according to Claim 1 or 2, wherein the halide of the substituting compound is present in a quantity, expressed in moles, which corresponds to the desired multiplicity of substitution with that compound.

4. Process according to any one of Claims 1-3, wherein the halide is a bromide.

5. Process according to any one of Claims 1-4, wherein the substituting compound is a primary or secondary alkyl halide.

6. Process according to any one of Claims 1-5, wherein the base is an alkali metal hydroxide.

7. Process according to any one of Claims 1-6, wherein the base is NaOH.

8. Process according to any one of Claims 1-7, wherein the aqueous solution of the base is renewed intermediately.

## Patentansprüche

1. Verfahren zur Herstellung einer substituierten Cyclopentadien-Verbindung, welches Verfahren aus dem Umsetzen eines Halogenids einer substituierenden Verbindung in einer Mischung der Cyclopentadien-Verbindung und einer wässerigen Lösung einer Base in Anwesenheit eines Phasentransfer-Katalysators besteht, dadurch gekennzeichnet, daß während der Reaktion die Menge an Base relativ zur Cyclopentadien-Verbindung zu jedem Zeitpunkt zwischen 5 und 30 mol/mol beträgt.

2. Verfahren nach Anspruch 1, bei welchem die Menge an Base relativ zur Cyclopentadien-Verbindung zwischen 7 und 15 mol/mol beträgt.

3. Verfahren nach Anspruch 1 oder 2, bei welchem das Halogenid der substituierenden Verbindung in einer Menge, ausgedrückt in mol, vorliegt, die der gewünschten Multiplizität der Substitution mit dieser Verbindung entspricht.

4. Verfahren nach einem der Ansprüche 1 bis 3, bei welchem das Halogenid ein Bromid ist.

5. Verfahren nach einem der Ansprüche 1 bis 4, bei welchem die substituierende Verbindung ein primäres oder sekundäres Alkylhalogenid ist.

6. Verfahren nach einem der Ansprüche 1 bis 5, bei welchem die Base ein Alkalimetallhydroxid ist.

7. Verfahren nach einem der Ansprüche 1 bis 6, bei welchem die Base NaOH ist.

8. Verfahren nach einem der Ansprüche 1 bis 7, bei welchem die wässerige Lösung der Base zwischendurch erneuert wird.

## Revendications

1. Procédé pour la préparation d'un composé de cyclopentadiène substitué, ce procédé consistant à faire réagir un halogénure d'un composé substituant dans un mélange du composé de cyclopentadiène et d'une solution aqueuse d'une base en présence d'un catalyseur de transfert de phase, caractérisé en ce que, au cours de la réaction, la quantité de base par rapport au composé de cyclopentadiène est à tout moment comprise entre 5 et 30 mol/mol.

2. Procédé selon la revendication 1, dans lequel la quantité de base par rapport aux composés de cyclopentadiène est comprise entre 7 et 15 mol/mol.

3. Procédé selon la revendication 1 ou 2, dans lequel l'halogénure du composé substituant est présent en une quantité, exprimée en moles, qui correspond à la multiplicité désirée de substitution avec ce composé.

4. Procédé selon l'une quelconque des revendications 1-3, dans lequel l'halogénure est un bromure.

5. Procédé selon l'une quelconque des revendications 1-4, dans lequel le composé substituant est un halogénure d'alkyle primaire ou secondaire.

6. Procédé selon l'une quelconque des revendications 1-5, dans lequel la base est un hydroxyde de métal alcalin.

7. Procédé selon l'une quelconque des revendications 1-6, dans lequel la base est NaOH.

8. Procédé selon l'une quelconque des revendications 1-7, dans lequel la solution aqueuse de base est renouvelée dans une étape intermédiaire.
